# FASCICULE DE BREVET EUROPEEN

(11) **EP 3 986 483 B1**
(45) Date de publication et mention de la délivrance du brevet: **09.10.2024**
(21) Numéro de dépôt: 20743776.5
(22) Date de dépôt: 16.06.2020
(51) Int. Cl.: A61L 2/00, A61L 2/18, A61L 2/24, A01N 53/00, B60P 3/00, B60P 3/04, B60P 3/14, E04H 1/12, E04B 1/343, E04H 15/06

(54) **UNITÉ MOBILE POUR LA DÉCONTAMINATION APPROFONDIE DE PERSONNES PAR VOIE HUMIDE**
MOBILE EINHEIT ZUR GRÜNDLICHEN NASSDEKONTAMINATION VON PERSONEN
MOBILE UNIT FOR THOROUGH WET DECONTAMINATION OF PERSONS

(30) Priorité: 21.06.2019 FR 1906703
(43) Date de publication de la demande: 27.04.2022
(73) Titulaire: Utilis, 57365 Ennery (FR)
(72) Inventeur: PREVOST, Philippe, 57000 Metz (FR)
(74) Mandataire: Radzimski, Eric
(86) Numéro de dépôt international: PCT/IB2020/055609
(87) Numéro de publication internationale: WO 2020/254955

(56) Documents cités:
- WO-A1-2007/144490
- CN-U- 205 706 344
- US-A1- 2003 037 812
- US-A1- 2005 053 533

## Description

### Domaine technique

La présente invention se rapporte de manière générale à la décontamination des personnes, par exemple suite à un incident par lequel une matière dangereuse est transférée de sa source vers des personnes. Elle concerne plus particulièrement une unité mobile de décontamination (UMD), pour la décontamination approfondie de personnes par voie humide.

### Etat de la technique

Par décontamination de personnes, on entend un procédé physique et/ou chimique de réduction, voire de suppression, et de prévention de la dispersion de la contamination des personnes. Concernant le risque de dispersion, on sait en effet que des personnes, des animaux, l'environnement ou des équipements, peuvent servir de vecteurs de transmission de la contamination, et c'est pourquoi il est souhaitable de décontaminer les personnes qui ont subi une contamination ou ont été simplement exposées à un risque de contamination. Une décontamination peut être réalisée sur le site d'un incident impliquant un agent contaminant. Elle peut aussi être réalisée à l'entrée de l'hôpital lorsque des évacuations de personnes depuis le site de l'accident vers un hôpital ont eu lieu de manière non contrôlée.

Dans le contexte de l'invention, un agent ou produit contaminant (aussi appelé « contaminant » par raccourci) désigne toute matière dangereuse à caractère peu volatil, persistant et toxique en faible quantité, qui se maintient physiquement et/ou chimiquement sur les personnes, et qui est susceptible de générer une intoxication en cas d'exposition.

La décontamination dans le cadre du NRBC (nucléaire, radiologique, biologique ou chimique), par exemple, commence par un tri de la population concernée. On sépare ainsi, le cas échéant, les personnes asymptomatiques (As), les personnes symptomatiques valides (SV), et les personnes symptomatiques invalides (SI).

On procède ensuite à une décontamination primaire, ou décontamination d'urgence, suivie éventuellement mais non systématiquement de la mise en oeuvre d'une décontamination secondaire, ou décontamination approfondie, permettant une décontamination fine dans le cadre d'une chaîne de décontamination.

La décontamination d'urgence a pour objectifs de réduire la contamination sur les victimes, et de limiter au maximum le transfert d'agents liquides et/ou solides, éventuellement volatils, vers les personnels et les matériels de secours. Cette procédure vise également à réduire les risques d'intoxication liés à la désorption d'agents contaminants à partir des vêtements. Elle se décompose comme suit :
- la décontamination sèche : ab(ad)sorption et déplacement des agents toxiques présents sur la surface corporelle (à l'aide de terre à foulon ou d'autres produits absorbants comme du tissu/papier absorbant,...) ;
- le déshabillage au minimum des couches superficielles (extérieures) de vêtements et les chaussures ; et,
- le rhabillage avec un kit (pyjama ou équivalent) si la victime est orientée vers le centre d'accueil des impliqués (CADI) ou avec une couverture de survie si elle est orientée vers une décontamination approfondie (voir ci-dessous).
En situation dégradée et en fonction des ressources disponibles, il est possible de procéder, après déshabillage, à une décontamination par transfert en utilisant l'effet mécanique de l'eau soit au moyen d'un tunnel hydraulique (tunnel d'engins d'incendie assurant un déluge d'eau) soit avec des douches d'infrastructures disponibles à proximité immédiate (gymnase, piscine, etc.).

La décontamination approfondie (ou décontamination « humide »), quant à elle, vise à éliminer les agents contaminants encore présents sur la surface corporelle et mobilisables par déplacement sous l'action mécanique de l'eau. A cet effet, elle se décompose comme suit :
- déshabillage complet des victimes ;
- lavage par douche à l'eau additionnée d'un tensioactif de type savon liquide, comme le savon liquide chirurgical (environ 0,5%), d'une durée déterminée, de l'ordre de 2 minutes ; et,
- rinçage par douche à l'eau claire d'une durée déterminée, de l'ordre de 2 minutes comme pour le lavage.
Les personnes avec des cheveux longs peuvent suivre deux cycles de douche. Le lavage peut être optimisé avec un gant de toilette à usage unique. Ainsi, la prise en charge des victimes à l'issue de la décontamination (soins, évacuation) peut se faire sans équipement de protection spécifique. On notera que la décontamination approfondie n'est pas systématique. Elle peut, après une phase initiale d'incertitude, être remise en question en fonction du contexte d'exposition (cas des gaz ou agents très volatils, ...).

L'efficacité de la décontamination d'urgence est d'autant plus importante qu'elle est effectuée le plus rapidement possible après l'exposition aux agents toxiques. Cela implique qu'elle doit idéalement être effectuées en urgence, directement sur le site de la contamination où les corps ont été exposés à des produits contaminants. C'est pourquoi les forces de secours, comme les sapeurs-pompiers, ou les forces militaires qui peuvent parfois être sollicitées même en cas de sinistre purement civil comme un accident industriel par exemple, disposent de matériels mobiles pour effectuer des opérations de décontamination d'urgence. De tels matériels mobiles peuvent en effet être projetés rapidement sur le lieu du sinistre.

Inversement, les matériels de décontamination approfondie sont des installations fixes positionnées de manière permanente dans des endroits prédéterminés, notamment sur des sites potentiellement dangereux. Leur localisation est définie en fonction d'informations recueillies préalablement à tout évènement de contamination, dans le cadre d'une analyse de risque, par exemple une analyse du risque industriel. Il s'ensuit que, en cas de sinistre, l'installation pourra se trouver trop près ou trop loin du lieu du besoin. L'invention trouve des applications, en particulier, dans la décontamination approfondie des victimes valides ou invalides, par voie humide. Plus particulièrement, l'invention vise à proposer une unité mobile pour la décontamination approfondie de personnes. Etant mobile contrairement aux installations de décontamination approfondie de l'art antérieur, elle peut se déplacer après la survenue d'un évènement contaminant afin d'être utilisée au plus proche de la zone contaminée. L'unité permet donc un positionnement du moyen de décontamination par voie humide immédiatement et à l'endroit exact du besoin. Dans ce contexte, l'homme du métier appréciera que l'unité mobile de décontamination approfondie qui est proposée permet une décontamination approfondie, par voie humide, là où l'état de l'art ne permet qu'une décontamination d'urgence, qui est généralement réalisée par voie sèche uniquement sauf à pouvoir utiliser un tunnel d'engins d'incendie assurant un déluge d'eau ou des douches d'infrastructures disponibles à proximité immédiate comme il a été exposé plus haut.

On rappelle que la décontamination par voie humide désigne toute technique de décontamination produisant un effet de dissolution du produit contaminant et de transfert dudit contaminant du porteur vers les effluents. La décontamination par lavage ou douchage à l'eau seule, par exemple, est déjà une excellente méthode de décontamination de masse. Le lavage à l'eau additionnée d'un agent dissolvant (comme du savon chirurgical à 0,5%), suivi par un rinçage du corps des victimes, améliore l'élimination du contaminant. Mais cela augmente la durée du processus de décontamination de chaque victime. La décontamination par voie humide est un procédé long, et consommateur de main-d'oeuvre.

Or, les moyens matériels et humains à disposition sont, la plupart du temps, très contraints. La réalisation des gestes de secours et de sauvetage, puis la première prise en charge médicale, suivies du pré-tri entre victimes valides ou invalides, puis ensuite l'évacuation vers l'hôpital, sont autant d'opérations à effectuer qui mobilisent les moyens matériels et humains des forces de secours.

En outre, le site de l'incident à l'origine de la contamination devient rapidement le théâtre d'une activité très intense après l'arrivée des forces de secours, en raison de la multitude des opérations mentionnées ci-dessus qui doivent y être réalisées simultanément et dans l'urgence. La gestion de l'espace, et les flux de personnes y deviennent très vite des problématiques de première importance pour l'efficacité globale des secours, surtout lorsque le nombre des victimes est élevé.

Pour toutes ces raisons, le besoin existe d'une unité de décontamination mobile pour la décontamination de personnes par voie humide, qui permette d'assurer un débit élevé des personnes décontaminées et qui structure et rationnalise les flux de personnes à proximité de l'unité de décontamination afin de minimiser l'impact des opérations de décontamination sur l'efficacité des autres opérations (opérations de secours, de prise en charge médicale, de tri entre victimes valides et invalides, et d'évacuation des victimes, notamment).

### Résumé de l'invention

L'invention vise à supprimer, ou du moins atténuer, tout ou partie des inconvénients de l'art antérieur précités.

A cet effet, un premier aspect de l'invention propose une unité de décontamination mobile pour la décontamination de personnes par voie humide, ayant la forme générale d'un parallélépipède rectangle avec un axe longitudinal et un axe transversal, caractérisée en ce qu'elle comprend :
- un module technique et au moins un module de douche, adjacents l'un à l'autre suivant la direction de l'axe longitudinal ;
- une première extension escamotable du module de douches suivant la direction de l'axe transversal, d'un côté dudit module de douche, et une seconde extension escamotable du module de douche suivant ladite direction de l'axe transversal, de l'autre côté dudit module de douche, les extensions sont formées de toiles en PVC, le module de douche ainsi que sa première extension et sa seconde extension comprenant au moins une ligne de décontamination de personnes pour la circulation, suivant la direction de l'axe transversal, de personnes à décontaminer par douchage dans le module de douche, dans laquelle le module de douche est équipé d'une douche de décontamination et d'une douche de rinçage pour chaque ligne de décontamination, comprenant chacune un pommeau de douche avec un circuit d'alimentation alimentée par une solution de décontamination et avec un autre circuit d'alimentation alimenté par de l'eau claire, respectivement.

Dans un mode de réalisation, le module de douche ainsi que sa première extension et/ou sa seconde extension comprennent au moins deux lignes de décontamination de victimes valides, qui sont adaptées pour être opérées en parallèle avec des moyens techniques communs compris dans le module technique.

Dans un mode de réalisation, les lignes de décontamination peuvent être adaptées pour être opérées en parallèle mais de manière indépendante l'une de l'autre.

Dans un mode de réalisation, le module de douche peut comprendre une paroi amovible s'étendant suivant la direction de l'axe transversal afin de séparer deux compartiments de douche du module de douche, lesdits compartiments de douche appartenant à la première ligne de décontamination et à la seconde ligne de décontamination, respectivement, et la première extension et/ou la seconde extension du module de douche peuvent alors comprendre chacune une paroi s'étendant suivant la direction de l'axe transversal afin de séparer ladite extension en deux compartiments, lesdits compartiments appartenant à la première ligne de décontamination et à la seconde ligne de décontamination, respectivement.

Dans un mode de réalisation, la première et la seconde extension du module de douche forment un compartiment de déshabillage des personnes et un compartiment de rhabillage des personnes, respectivement.

L'unité de décontamination peut comprendre un unique bac tampon qui est placé sous un plancher en caillebotis du module de douche, et elle peut comprendre en outre une pompe qui est placée dans le module technique et qui est adaptée pour aspirer les eaux usées dans le bac tampon et les évacuer vers un ou plusieurs réservoirs souples de récupération des eaux usées qui peuvent être placés à l'extérieur de l'unité de décontamination.

Dans un mode de réalisation, le fond du bac tampon peut avoir un profil en V, avec une pente dirigée vers la pompe.

Dans un mode de réalisation, la première extension et/ou la seconde extension du module de couche sont en toile, et sont dépliables et repliables avec des éléments d'armature et des poteaux par lesquels la toile est soutenue, ladite toile, lesdits éléments d'armature est lesdits poteux étant escamotables dans la paroi du module de douche.

Dans un mode de réalisation le module technique peut comprendre, en outre, un ventilateur raccordé à une gaine de diffusion d'air débouchant dans la seconde extension, et adapté pour assurer un débit d'air assurant un balayage d'air en surpression depuis la seconde extension vers la première extension à travers le module de douche.

Dans un mode de réalisation, le fonctionnement du module de douches de l'unité peut être automatique, et rythmé par des indicateurs lumineux placé au-dessus de la porte d'entrée, de la porte de sortie et au milieu des deux compartiments de douche, la lumière s'allumant rouge ou verte en fonction du fonctionnement des douches.

Dans un mode de réalisation, le module de douche ainsi que sa première extension et/ou sa seconde extension, peuvent comprendre au moins une ligne de décontamination de victimes invalides, qui est adaptée pour être opérée, en mode dégradé, en tant que ligne de décontamination de personnes valides.

Dans un mode de réalisation, la ligne de décontamination pour personnes invalides comprend :
- des supports hauts avec des rails de brancardage disposés dans la première extension et dans la seconde extension ;
- une table élévatrice à deux positions basse et haute, disposée dans le module de douche, la position basse de la table élévatrice venant de niveau avec les rails de brancardage de la première extension et avec les rails de brancardage de la seconde extension, la table élévatrice venant en alignement opérationnel avec lesdits rails de brancardage de manière à former une ligne de brancardage suivant la direction de l'axe transversal Y.

Dans un mode de réalisation, l'unité de décontamination comprend au moins un brancard pour faire passer une personne invalide le long de la ligne de décontamination pour personnes invalides, dont la toile est perforée afin que l'eau puisse s'écouler à travers.

Dans un second aspect, l'invention concerne également un procédé d'utilisation d'une unité de décontamination selon l'aspect comprenant des indicateurs lumineux ci-dessus, dans lequel le fonctionnement de douches du module de douche est automatique, et est commandé en rythme avec l'allumage d'indicateurs lumineux adaptés pour signaler aux personnes valides leur progression dans la ligne de décontamination pour personnes valides.

### Brève description des figures

D'autres caractéristiques et avantages de l'invention apparaîtront encore à la lecture de la description qui va suivre. Celle-ci est purement illustrative et doit être lue en regard des dessins annexés sur lesquels :
**[****Fig. 1****]** est une représentation schématique de la forme générale de l'unité de décontamination conforme à des modes de réalisation ;
[Fig. 2] est une vue en trois dimensions de l'unité de décontamination montée sur un véhicule porteur, selon une version de l'unité de décontamination équipée d'un module de douche adaptée pour les personnes valides ;
[Fig. 3] est une autre vue de l'unité de décontamination de la figure 1 ;
[Fig. 4] est une vue en trois dimensions d'une extension latérale du module de douche de l'unité de douche des figures 2 et 3, formant un sas d'entrée pour le déshabillage des personnes ;
[Fig. 5] est une vue en trois dimensions, partielle, d'une autre extension latérale du module de douche de l'unité de douche des figures 2 et 3, formant un sas de sortie pour le séchage/rhabillage des personnes, montrant une gaine de diffusion d'air débouchant dans le sas de sortie ;
**[****Fig. 6****]** est une vue de dessus de l'unité de décontamination pour personnes valides des figures 2 et 3 ;
**[****Fig. 7****]** est une vue de côté droit de l'unité de décontamination pour personnes valides des figures 2 et 3 ;
**[****Fig. 8****]** est une vue de côté gauche de l'unité de décontamination pour personnes valides des figures 2 et 3 ;
**[****Fig. 9****]** est une vue de dessus d'une unité de décontamination équipées d'un module de douche pour personnes invalides des figures 2 et 3 ;
**[****Fig. 10****]** est une vue de côté droit de l'unité de décontamination pour personnes invalides de la figure 9 ;
**[****Fig. 11****]** est une vue de côté gauche de l'unité de décontamination pour personnes invalides de la figure 9 ;
**[****Fig. 12****]** est une vue du côté de l'unité de décontamination montrant des moyens d'évacuation des eaux usées ;
**[****Fig. 13****]** est une vue d'une pompe faisant partie des moyens d'évacuation des eaux usée de la figure 12 ;
**[****Fig. 14****]** est une vue d'un réservoir d'eau propre de l'unité de décontamination ;
**[****Fig. 15****]** est une représentation d'une partie des moyens d'alimentation en eau des douches de l'unité de décontamination ; et,
**[****Fig. 16****]** est une représentation d'un élément du circuit d'alimentation en eau de l'unité de décontamination.

### Description des modes de réalisation

Dans la description de modes de réalisation qui va suivre et dans les Figures des dessins annexés, les mêmes éléments ou des éléments similaires portent les mêmes références numériques aux dessins.

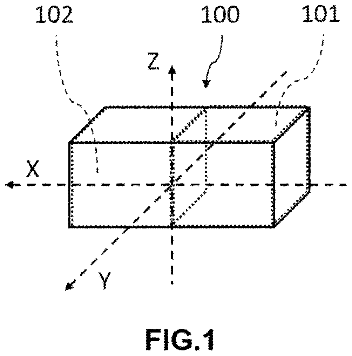

En référence à la **figure 1****,** une unité de décontamination mobile conforme aux modes de réalisation est une cellule 100 qui affecte la forme générale d'un parallélépipède rectangle, avec un axe longitudinal X, un axe transversal Y, et un axe vertical Z. Un parallélépipède rectangle est une figure solide délimitée par six faces rectangulaires (boîte rectangulaire), dont tous les angles sont des angles droits et dont les faces opposées sont égales.

En pratique, la cellule 100 peut être installée de manière fixe ou transférable sur le châssis d'un véhicule porteur.

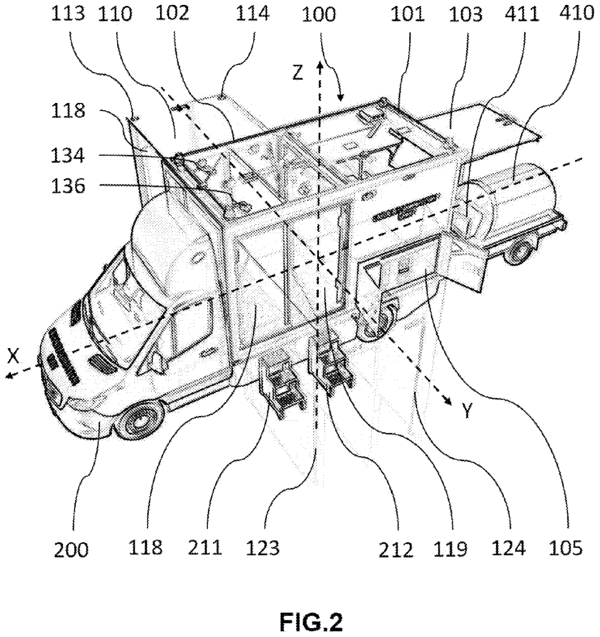

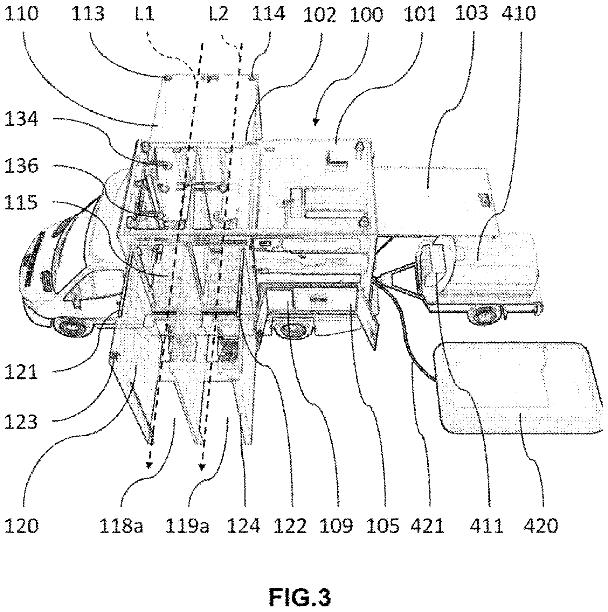

Dans le cas non limitatif des modes de réalisation envisagés dans la présente description et représentés notamment sur la **figure 2** et la **figure 3****,** un tel véhicule porteur 200 est, par exemple, un châssis porteur de type Plancher-Cabine de marque MERCEDES@. Plus particulièrement, il peut s'agir d'un véhicule de type Sprinter^{®} 519 CDI, en version poids lourd de 5 Tonnes, équipé par exemple d'un moteur de 190 CV à 6 cylindres développant 2987 cc de cylindrée et offrant un couple de 440 Nm à 1400-2400 tr/mn, ainsi que d'une boite de vitesse automatique.

Dans une telle installation, l'axe longitudinal X de la cellule 100 coïncide avec l'axe longitudinal du véhicule porteur 200, et est orienté vers l'avant dudit véhicule en référence au sens de déplacement du véhicule en marche normale ou marche-avant. C'est pourquoi, par convention et sauf mention contraire expresse, les termes « avant » et « arrière » ainsi que leurs dérivés « en-avant » et « en-arrière », ou « vers l'avant » et « vers l'arrière », ainsi que les termes « devant » et « derrière », sont utilisés dans ce qui suit en référence à la direction de l'axe longitudinal X du véhicule porteur 200, orienté vers l'avant du véhicule.

Par ailleurs, l'axe transversal Y de la cellule 100 coïncide avec l'axe longitudinal du véhicule porteur 200. Les termes « côté » et « latéral » et leurs dérivés, et ainsi que les termes « gauche » et « droite » et leurs dérivés « à gauche » et « à droite », ou « vers la gauche » et « vers la droite », sont utilisés dans ce qui suit en référence à la direction de l'axe transversal du véhicule porteur 200 et du point de vue d'un observateur regardant vers l'avant du véhicule.

Enfin, l'homme du métier appréciera que la direction de l'axe vertical Z de la cellule 100 ou du véhicule porteur 200 correspond à la direction de la gravité. C'est pourquoi, sauf mention contraire expresse, les termes « haut » et « bas » ainsi que leurs dérivés « en-haut » et « en-bas », ou « vers le haut » et « vers le bas », ainsi que les termes « supérieur(e) » et « inférieur(e) » et que les termes « dessus » et « dessous » et leurs dérivés « au-dessus » ou « en-dessous » et « par-dessus » ou « par-dessous », sont utilisés dans ce qui suit en référence à la direction de l'axe vertical Z.

Toujours en référence à la figure 1, et comme montré notamment sur les figures 2 et 3, la cellule 100 de l'unité de décontamination selon des modes de réalisation comprend deux modules 101 et 102, adjacents l'un à l'autre suivant la direction de l'axe longitudinal X. Le module 101 est un module technique, et le module 102 est un module de douche adapté pour la décontamination des personnes par voie humide.

Le module technique 101 regroupe l'ensemble des équipements et des organes de commande nécessaires au fonctionnement de l'unité de décontamination 100. Dans l'exemple représenté, le module technique 101 est placé à l'arrière de la cellule 100. Son accès se fait alors avantageusement par l'arrière du véhicule 200, par l'intermédiaire d'une porte arrière 103, par exemple un hayon s'ouvrant vers le haut. Ainsi qu'il apparaîtra dans la suite du présent exposé, l'accès au module technique 101 par un opérateur 300 peut ainsi se faire sans gêne mutuelle avec la circulation des personnes dans la ou les lignes de décontamination L1 et L2, ou L3, qui passent transversalement à travers le module de douche 102.

Du fait de la conception et de l'agencement du module de douche 102 à l'avant du véhicule 200, par exemple juste derrière la cabine du véhicule, toute la zone extérieure située à l'arrière du véhicule 200 est en effet libre de toute circulation des personnes à décontaminer. Cette zone peut être organisée par les forces de secours lors du déploiement de l'unité de décontamination, avec un accès réservé aux opérateurs de l'unité de décontamination pour leur permettre de réaliser des opérations techniques. Ces opérations comprennent par exemple le remplissage ou le remplacement d'une remorque du camion 200 comportant une citerne 410 qui est raccordée au module technique 101, et/ou la mise en place et l'installation ou au contraire l'enlèvement et/ou le remplacement d'un réservoir 420 de collecte des eaux usées qui est lui-aussi raccordé au module technique 101. Cette citerne 410 et ce réservoir 420 sont en effet couplés au module technique 10 pour permettre son fonctionnement opérationnel, en sorte que les opérations précitées sont facilitées du fait que ledit module est à l'arrière du véhicule 200. Ces opérations nécessitent en général l'approche d'un autre véhicule, par exemple pour déposer un opérateur au plus près du module technique et ainsi réduire son exposition aux éventuelles retombées ambiantes de produit contaminant sur le site de l'intervention, et/ou pour emporter la citerne 410 lorsqu'elle est vide, et/ou pour déposer une citerne de remplacement qui est pleine, ou encore pour emporter le réservoir 420 d'eaux usées lorsqu'il est plein et en déposer un autre à la place. Il est donc avantageux que la citerne 410 et le réservoir 420 puissent être disposés dans la zone à l'arrière du véhicule 200 et du module technique 110, lequel est lui-même disposé à l'arrière du véhicule à cet effet et à l'effet de l'accès par des opérateur par l'arrière du véhicule 200, alors que la circulation des personnes à travers le module de douche 102 se fait transversalement d'un côté à l'autre de l'unité de décontamination 100 et donc du véhicule 200.

Comme on l'aura compris, en effet, le flux des personnes à décontaminer ne passe pas par la zone à l'arrière de l'unité 100 et donc du camion porteur 200, si bien qu'il n'y a aucune gêne mutuelle. La circulation des personnes à décontaminer est donc fluide et rapide, et les opérations d'accès au module technique 101 par l'opérateur et/ou les opérations en rapport avec les équipements qui lui sont raccordés comme la citerne 410 et le réservoir 420 sont facilitées car les autres véhicules et/ou les opérateurs impliqués par ces opérations peuvent s'approcher de l'unité de décontamination 100 sans risque pour, et sans gêne par lesdites personnes.

Un autre avantage de l'agencement du module technique 101 à l'arrière de la cellule 100 et donc du véhicule 200, est que le hayon 103 permet en outre, lorsqu'il est en position relevée, de protéger l'opérateur 300 et le matériel qu'il utilise contre la pluie et contre d'éventuelles retombées de produits contaminants flottant dans l'espace ambiant, lorsque l'opérateur 300 descend dudit module technique 101 pour intervenir sur la citerne 4410 et/ou sur le réservoir 420.

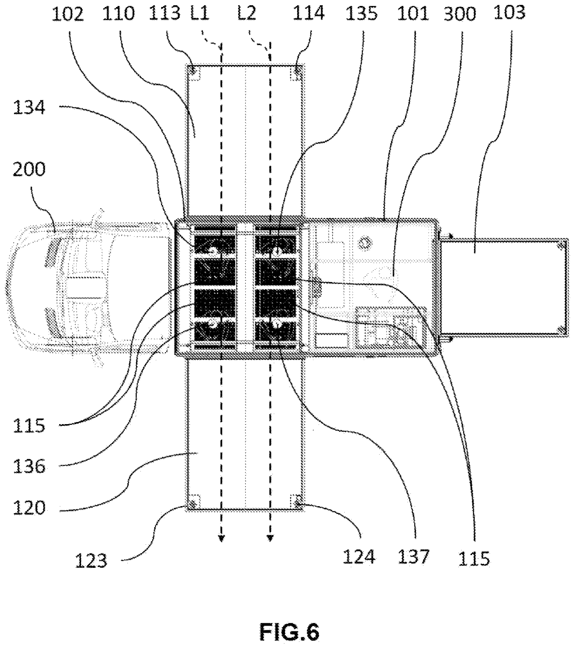

On va maintenant décrire de manière générale les lignes de décontamination L1 et L2 (dans le cas de l'unité de décontamination pour personnes valides conforme aux modes de réalisation illustrés sur les figures 6, 7 et 8), ou la ligne de décontamination L3 (dans le cas de l'unité de décontamination pour personnes invalides conformes aux modes de réalisation illustrés sur les figures 6, 7 et 8). En bref, les lignes de décontamination suivies par les personnes à décontaminer s'étendent transversalement à l'unité 100 suivant la direction de l'axe Y. Les personnes accèdent à, et sortent du module de douche 102 par des portes latérales dudit module 102 prévues à gauche et à droite de ce module, respectivement.

Dans l'exemple d'unité de décontamination pour personnes valides qui est montré aux figure 2, 3, 6, 7 et 8, l'unité 100 comprend deux lignes de décontamination L1 et L2, qui sont adaptées pour être opérées en parallèle l'une de l'autre. Ces lignes L1 et L2 s'étendent parallèlement l'une à l'autre suivant la direction transversale Y. Le module de douche 102 comprend donc deux compartiments de douche, à savoir un tel compartiment pour chacune des lignes de décontamination L1 et L2, respectivement.

Les dimensions intérieures d'un compartiment du module de douche 102 sont par exemple égales à 2000 x 900 mm, c'est-à-dire 2000 mm de long (suivant la direction transversale Y de l'unité 100 et du véhicule 200, entre le côté droit et le côté gauche dudit véhicule), sur 900 mm de large (suivant la direction longitudinale X de l'unité 100).

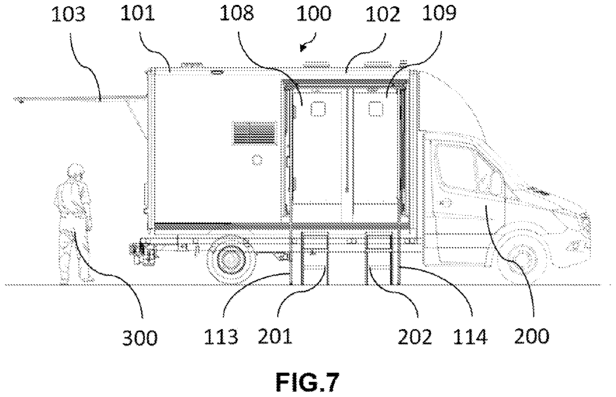

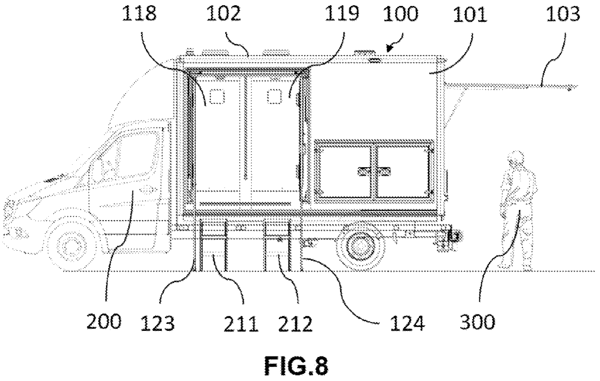

En référence notamment aux figures 7 et 8, l'entrée et la sortie du compartiment de douche 102 se font par le biais de portes rigides dudit compartiment 102. Ces portes sont référencées 108 et 109 à la figure 7 pour les portes d'entrée de chacune des lignes de décontamination L1 et L2, situées du côté droit de l'unité 102. Et elles sont référencées 118 et 119 à la figure 8 pour les portes de sortie de chacune des lignes de décontamination L1 et L2, situées du côté droit de l'unité 102. Les portes rigides 108, 109, 118 et 119 sont par exemple en matériau stratifié compact. Une porte réalisée dans un tel matériau inerte a l'avantage de ne pas réagir avec les variations de température et d'humidité, réduisant le risque de défaut d'étanchéité du module de douche pendant son utilisation. Les vapeurs de douche, potentiellement toxiques, restent donc bien confinées dans le compartiment de douche 102.

Dans un mode de réalisation, les portes 108, 109, 118 et 119 peuvent être sont équipées d'un ressort de rappel garantissant sa fermeture après chaque passage d'une victime, toujours dans le but d'éviter la dispersion de vapeurs de douche potentiellement toxiques vers l'extérieur du module de douche 102, notamment du côté du sas de sortie 120 dont la propreté et l'innocuité doivent être préservées.

Les portes 108, 109, 118 et 119 peuvent aussi être équipées d'un oculus de dimensions égales à 120 x 120 mm, par exemple, surtout du côté de l'entrée (côté droit de l'unité 100). Ces oculi peuvent être disposé à environ 1500 mm du sol du module de douche 102. Ainsi disposés, ils permettent à une personne située à l'extérieur du module de douche 102 sur les marches-pieds présentés dans ce qui précède, notamment les personnes suivantes dans chaque ligne de décontamination L1 et L2, de vérifier ce qui se passe dans les compartiments de douche afin par exemple d'anticiper leur entrée dans ledit module de douche.

Chacun des compartiments de douche du module de douche 102 comprend deux zones de douche adjacentes l'une à l'autre suivant la direction transversale Y de l'unité 100. Chaque zone a donc des dimensions d'environ 1000 x 900 mm, c'est-à-dire 1000 mm de long (suivant la direction transversale Y), sur 900 mm de large (suivant la direction longitudinale X). Chaque zone est équipée d'un pommeau de douche. Le premier, pour la zone du compartiment de douche du côté de l'entrée (côté gauche dans l'exemple considéré ici) porte, sur les figures des dessins annexés, la référence 134 pour la ligne L1 et la référence 135 pour la ligne L2. Le second, pour la zone du compartiment de douche du côté de la sortie (côté droit du camion 200 dans l'exemple considéré ici), porte la référence 136 pour la ligne L1 et la référence 137 pour la ligne L2. La hauteur entre le sol du module de douche 102 et chaque pommeau de douche 134, 135, 136 et 137 est d'environ 2000 mm.

L'alimentation en eau desdits premiers pommeaux de douche 134 et 135 et desdits seconds pommeaux de douche 136 et 137 se fait par deux circuits respectifs, qui sont indépendants l'un de l'autre. Un premier circuit pour le lavage (décontamination) alimente les douches 134 et 135, et un second circuit pour le rinçage alimente les douches 136 et 137. Le premier circuit est connecté à une pompe mélangeuse afin d'additionner un produit de lavage à l'eau. Le second circuit pour le rinçage est alimenté par de l'eau claire.

Dans l'exemple d'un module de douche pour personnes valides avec deux lignes L1 et L2 parallèles qui est représenté notamment à la figure 6, le module de douche peut accueillir simultanément quatre personnes, à raison de une personne sous chacune des douches 134, 135, 136 et 137, respectivement. De préférence, toutefois, chaque compartiment de douche respectivement associé à l'une des lignes de décontamination L1 et L2 (et qui comprend la zone sous la douche de lavage 134 ou 135, respectivement, ainsi que la zone sous la douche de rinçage 136 ou 137, respectivement) n'accueille qu'une seule personne à la fois. La personne se positionne tout d'abord dans la zone sous la douche de lavage, et y reçoit une douche de lavage, avant d'avancer dans la zone sous la douche de rinçage, pour y recevoir une douche de rinçage.

Dans tous les cas, et comme montré à la figure 6, deux personnes circulent en parallèle l'une de l'autre, au sein de la ligne L1 et de la ligne L2, respectivement. En même temps, deux autres personnes circulent derrière ces deux personnes, respectivement. Et deux autres personnes les précèdent au sein de ces deux lignes, respectivement

Un extracteur d'air équipé d'un filtre Très Haute Efficacité (THE) peut être disposée au-dessus des douches, afin notamment d'éviter que l'air dans la zone de décontamination comprenant les douches de lavage 134 et 135 (du côté de l'entrée du module de douche 102, à droite du véhicule 200) n'aille polluer l'air dans la zone de rinçage comprenant les douches de rinçage 136 et 137 (du côté de la sortie du module de douche 102, à gauche du véhicule 200). Le débit d'aspiration est par exemple de 3000 m³/h. Le colmatage du filtre peut être contrôlé par un manomètre adapté pour mesurer la dépression en amont du filtre.

Au sein du module de douche 102, les compartiments de douche respectivement associés aux deux lianes de décontamination L1 et L2 sont séparés l'un de l'autre par un mur en toile PVC, par exemple en toile PVC ayant une densité de 500g/m². Ce mur peut être amovible, ce qui permet lorsqu'il est retiré de pouvoir faire passer ensemble une mère et son enfant, par exemple, en parallèle. La mise en place ou la dépose d'un tel mur amovible en toile ne prennent que très peu de temps.

Les murs et le plafond du module de douche 102 peuvent être réalisés en panneau sandwich dont les parements sont en Polyester non poreux, de préférence totalement lisse et donc facilement décontaminables.

Le sol du module de douche 102 peut être réalisé en caillebotis rigide 115, par exemple en Inox, posés sur des traverses également en Inox, ce qui le rend facilement déposable. La dépose des caillebotis permet aussi l'accès à un ou plusieurs bacs tampon de récupération des eaux usées disposés en-dessous, afin de les décontaminer après la fin de l'utilisation de la cellule de décontamination 100. Les caillebotis 115 sont de préférence anti-dérapants afin de prévenir le risque de glissage et de chute des personnes dans le module de douche.

Dans des modes de réalisation, le module de douche comprend avantageusement un seul et unique bac tampon placé sous les douches, qui permet de récupérer l'eau usée issue des quatre douches 134-137, et de diriger toute cette eau vers la pompe de récupération des eaux usées. A cet effet, le bac de récupération des eaux usées comprend une pente vers une zone d'aspiration par une pompe d'évacuation des eaux usées sur laquelle on reviendra plus loin.

Compte tenu de la hauteur de la cellule 100 par rapport au sol lorsqu'elle est montée sur le châssis du camion porteur 200, la montée et la descente du module de douche 102 peuvent se faire par des marches-pieds qui sont fournis pour accéder et sortir dudit compartiment de douche. Ces marches-pieds peuvent être des éléments séparés du module de douche 102, ou être des éléments escamotables depuis des logements ad-hoc prévus dans le châssis du camion porteur 200, par exemple.

En référence en particulier aux figures 7 et 8, il y a ainsi deux marches-pieds 201 et 202 du côté droit, à raison de un pour chaque ligne de décontamination L1 et L2, respectivement, prévus pour l'entrée (montée) dans le compartiment de douche respectif du module de douche 102. Et il y a pareillement deux marches-pieds 211 et 212 du côté gauche, à raison de un pour chaque ligne de décontamination L1 et L2, respectivement, prévus pour la sortie (descente) du compartiment de douche respectif du module de douche 102. Idéalement, et compte tenu en particulier du contexte humide, surtout du côté de la descente qui suit l'opération de douchage des personnes, c'est-à-dire à gauche de la cellule 100 dans l'exemple, les marches-pieds sont de préférence anti-dérapants.

Avantageusement, l'entrée et la sortie des personnes se font via un sas d'entrée et/ou via un sas de sortie qui sont adjacents au module de douche 102 suivant la direction transversale Y, à droite et à gauche de l'unité 100, respectivement. Ces sas peuvent être réalisés comme des extensions transversales 110 et 120 du module de douche. Avantageusement, ces extensions sont installées sur site uniquement, lorsque le camion porteur 200 est à l'arrêt et est stabilisé par des vérins, par exemple. De préférence, ces extensions 110 et 120 sont solidaires de la cellule 100, et sont escamotées dans ou contre les parois latérales de ladite cellule.

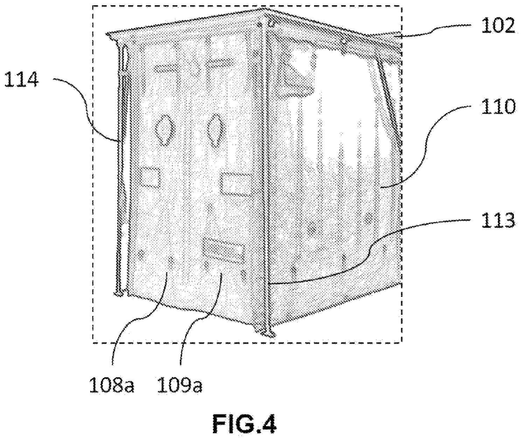

L'unité 100 comprend ainsi une première extension escamotable du module de douches 102 suivant la direction de l'axe transversal, d'un côté dudit module de douche. Dans l'exemple représenté aux figures et en particulier à la **figure 4****,** cette extension 110 est située du côté droit du module 102. Elle sert de sas d'entrée pour l'accès des personnes au module de douches 102. Du point de vue de l'utilisation, l'extension 110 est adaptée pour offrir un compartiment de déshabillage des personnes avant leur entrée dans le module de douche. Plus particulièrement, il y a dans l'extension 110 un tel compartiment pour chacune des lignes de décontamination fournies par l'unité de décontamination 100. On sait que le déshabillage des personnes contaminées représente à lui seul un enlèvement de plus de 80% des contaminants. Les vêtements contaminés sont par exemple déposés dans des corbeilles (non représentées) qui sont posées au sol ou suspendues dans le sas d'entrée 110, pour être enlevés et détruits, dans la plupart des cas d'usage de l'unité de décontamination 100.

On rappelle que, dans l'exemple d'unité de décontamination pour personnes valides qui est montré aux figure 2, 3, 6, 7 et 8, l'unité comprend deux lignes de décontamination L1 et L2, qui sont adaptées pour être opérées en parallèle l'une de l'autre. Ces lignes L1 et L2 s'étendent parallèlement l'une à l'autre suivant la direction transversale Y. L'extension 110 comprend donc deux compartiments de déshabillage, à savoir un tel compartiment pour chacune des lignes de décontamination L1 et L2, respectivement.

L'accès à chaque compartiment de déshabillage se fait par une porte qui, à la figure 4, est référencée 108a pour la ligne L1 et 108b pour la ligne L2, respectivement. Chaque porte peut être équipée de fermetures à glissière, et peut être maintenue en position haute.

Les dimensions de chaque compartiment respectivement associé à la ligne L1 et à la ligne L2 dans l'extension 110 formant le sas d'entrée, sont par exemple égales à 2000 x 900 mm, à savoir 2000 mm de long suivant la direction transversale Y, et 900 mm de large suivant la direction longitudinale X.

En variante ou en complément, l'unité 100 selon des modes de réalisation peut comprendre une seconde extension escamotable du module de douche 102 suivant ladite direction de l'axe transversal Y, de l'autre côté dudit module de douche. Dans l'exemple représenté, cette extension 120 est située du côté gauche du module 102. Elle sert de sas de sortie pour la sortie des personnes du module de douches 102. Du point de vue de l'utilisation, l'extension 120 est adaptée pour offrir un compartiment de séchage et de rhabillage des personnes après leur sortie du module de douche 102, dont l'environnement est humide.

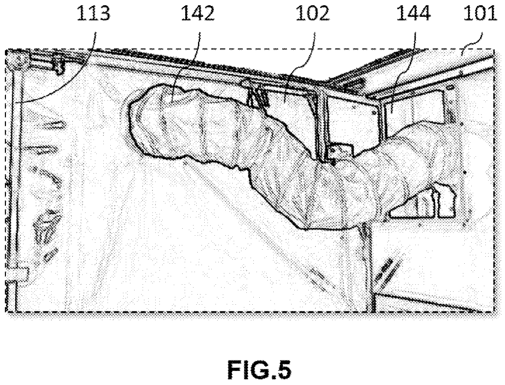

Dans un mode de réalisation illustré par la **figure 5****,** le module technique 101 comprend un ventilateur pour assurer un débit d'air, par exemple de 1500 m³/h, qui est raccordé à une gaine de diffusion d'air 142 débouchant dans le sas de rhabillage 120. Ce flux d'air assure un balayage d'air en surpression depuis la sortie vers l'entrée de l'unité mobile de décontamination. L'air potentiellement contaminé est donc reflué de la sortie vers l'entrée de la ligne de décontamination considérée, c'est-à-dire dans le sens inverse du sens de circulation des personnes le long des lignes de décontamination L1 et L2. Ceci évite, ou du moins réduit fortement le risque de dispersion des agents contaminants le long des lignes de décontamination, notamment lors de l'ouverture des portes rigides 108,109 et 118,119 du module de douche 102 qui pourrait autrement générer un courant d'air contaminé le long des lignes L1 et L2 à travers le module de douche. Dans un mode de réalisation, le module technique 101 comporte un logement 144 fermé par une trappe, dans lequel la gaine de diffusion d'air 142 est repliée et stockée en dehors des phases d'utilisation de l'unité de décontamination.

Le sas de rhabillage 120 peut être équipé, à l'intérieur, de sangles permettant le rangement des tenues à usage unique, que les personnes revêtent après leur décontamination afin de pouvoir rentrer chez elles.

De même que pour l'extension 110 formant le sas d'entrée 110, il y a dans l'extension 120 formant le sas de sortie un tel compartiment de rhabillage pour chacune des lignes de décontamination L1 et L2 fournies par l'unité de décontamination 100.

La sortie de chaque compartiment de rhabillage se fait par une porte qui, à la figure 3, est référencée 118a pour la ligne L1 et 119a pour la ligne L2, respectivement. Chaque porte peut être équipée de fermetures à glissière, et peut être maintenue en position haute.

Comme pour l'extension 110 formant le sas d'entrée, les dimensions de chaque compartiment respectivement associé à la ligne L1 et à la ligne L2 dans l'extension 120 formant le sas de sortie, sont par exemple égales à 2000 x 900 mm, à savoir 2000 mm de long suivant la direction transversale Y, et 900 mm de large suivant la direction longitudinale X.

Dans des modes de réalisation, les extensions 110 et 120 de la cellule 100 sont formées de toiles en PVC, par exemple de toiles en PVC ayant une densité de 500g/m². Les matériaux dont elles sont conçues peut être ignifugé, avec un classement M2 (difficilement inflammable) au classement défini dans la série de normes françaises NF P92-5XX (par exemple la norme NF P92-507), pour une utilisation sans risque sur des sites frappés par, ou sujets à un risque d'incendie.

Dans des modes de réalisation, en dehors des phases d'utilisation de la cellule de décontamination 100, les toiles restent pliées à demeure dans des coffres situés en haut du module de douche 102, de chacun des côtés droit et gauche pour les extensions 110 et 120, respectivement. Ceci permet une mise en place rapide de l'unité de décontamination par simple dépliage. Le temps de mise en service estimé de l'unité de décontamination est inférieur à 15mn, avec deux opérateurs.

En conditions d'utilisation, les extensions 110 et 120 dépliées de la cellule 100 peuvent être maintenues par des poteaux assurant le maintien de l'extension avec ses toiles. Ces poteaux 113 et 114 pour l'extension 110, et 123 et 124 pour l'extension 120, peuvent par exemple être en Inox. L'Inox est en effet un matériau inerte facile à décontaminer. Les toiles extérieures sont conçues pour pouvoir s'accrocher aux poteaux 113,114 et 123,124 par un système de bandes Velcro^{™} afin que les murs en toile des extensions 110 et 120 restent verticaux pendant l'utilisation de l'unité mobile 100. En configuration repliée des extensions 110 et 120, les poteaux 113,114 et 123,124 respectivement, sont repliés et intégrés à l'extension correspondante.

Les toiles sont solidaires de l'extension. Pour les déployer, il suffit d'ouvrir l'extension, de dérouler les toiles extérieures et de les relier dans les angles par un système de bandes Velcro^{™}. Les toiles peuvent avantageusement être marquées « Entrée » ou « Sortie », pour les extensions de chaque côté droit ou gauche, respectivement, de la cellule 100.

En résumé, chacune des lignes de décontamination L1 et L2 de l'unité de décontamination 100 comprend, pour la décontamination d'une personne, quatre postes ou stations dans lesquelles la personne passe successivement, à savoir :
- le compartiment de déshabillage dans le sas d'entrée 110 ;
- la zone de douche de lavage dans la première partie du module de douche (du côté de l'entrée 108 ou 109) ;
- la zone de douche de rinçage dans la seconde partie du module de douche (du côté de la sortie 118 ou 119) ; et,
- le compartiment de séchage et de rhabillage dans le sas de sortie 120.

A chacun des postes ci-dessus, la personne est l'objet et/ou est actrice d'une étape respective d'un procédé de mise en oeuvre de la cellule qui comprend quatre étapes successives pour la décontamination d'une personne par voie humide. Ces étapes sont, dans cet ordre, le déshabillage, la douche de lavage, la douche de rinçage, et le séchage et rhabillage de la personne.

A chacun des postes mentionnés plus haut et pour la réalisation de chacune des étapes ci-dessus, la personne est seule concernée dans un espace clos et à l'abri des regards des autres personnes. Dit autrement, elle est isolée des autres personnes formant un groupe de personnes à décontaminer puisque, à chaque fois, une seule personne à la fois se trouve, pour chacune des lignes L1 et L2, dans le compartiment de déshabillage du sas d'entrée 110, dans le compartiment de douche du module de douche 102, et dans le compartiment de séchage/rhabillage du sas de sortie 120.

Cela est avantageux du point de vue sanitaire. En effet, cela réduit le risque de contamination croisée entre les personnes au sein de l'unité de décontamination dans laquelle les espaces sont étroits et la promiscuité entre personnes est grande.

Cela est aussi un avantage du point de vue du respect de l'intimité des personnes, ce qui indirectement est favorable à la rapidité du processus de décontamination d'une personne, et donc aux performances de l'unité de décontamination en termes de débit du flux des personnes à décontaminer. En effet, à partir du compartiment de déshabillage (y-compris) dans le sas d'entrée 110, et jusqu'au compartiment de rhabillage (y-compris) dans le sas de sortie 120, chaque personne est isolée des autres personnes à décontaminer. De la sorte, aucun problème de pudeur n'affecte son comportement qui pourrait potentiellement freiner sa progression le long de la ligne de décontamination. Les personnes se sentent respectées, et donc coopèrent avec d'autant plus d'empressement.

Les avantages mentionnés dans ce qui précède proviennent aussi du fait que le compartiment de déshabillage et le compartiment de séchage/rhabillage sont contigus au module de douche 102. Les personnes n'ont pas à parcourir un trajet en espace libre entre des deux compartiments. Elles sont isolées des autres personnes à la fois du point de vue sanitaire et du point de vue humain et/ou éthique (respect de la personne humaine, des codes sociaux, de la morale, etc.).

Néanmoins, le fait qu'une personne puisse être en train d'être douchée dans le module de douche 102 pendant que la personne suivante est déjà en train de se préparer en se déshabillant dans le sas d'entrée 110, et pendant que la personne précédente achève le processus de décontamination la concernant en se rhabillant dans la sas de sortie 120, est favorable à un débit élevé du flux des personnes décontaminées. Bien entendu, la conception des lignes de décontamination selon les modes de réalisation proposés permet la disposition en parallèle de plusieurs lignes de décontamination comme les lignes L1 et L2. Plus il y a de lignes de décontamination, et plus le débit est important. Le module de commande étant commun aux lignes de décontamination, le coût de l'ajout d'une ligne de décontamination est marginal. La limite haute résulte du caractère limité des ressources consommables (énergie électrique, eau propre) dans le contexte d'une unité de décontamination mobile. Cela peut être une contrainte limitante si l'unité mobile n'a pas accès, sur le site de l'intervention, à un réseau électrique et/ou à un réseau d'alimentation en eau, ou à des ressources facilement renouvelées.

Dans l'exemple représenté aux figures 2, 3 6, 7 et 8, les deux lignes de décontamination L1 et L2 sont contigües l'une de l'autre afin d'optimiser l'espace disponible dans le module de douches et dans ses extensions 110 et 120. Les deux lignes de décontamination L1 et L2 sont séparées matériellement l'une de l'autre par des murs en toile PVC, par exemple en toile PVC ayant une densité de 500g/m². Un tel matériau offre l'avantage d'être facilement nettoyable, afin de permettre un réemploi. Également, c'est un matériau peu cher, en sorte que les murs en toile peuvent avantageusement être à usage unique. Avec des toiles opaques, on obtient une isolation visuelle entre les deux lignes L1 et L2, ce qui veut dire qu'une personne dans la ligne L1 ne peut pas voir ni ne peut être vue d'une autre personne se trouvant au même niveau dans la ligne L2. Les toiles assurant la séparation intérieure des compartiments respectifs dans le sas de déshabillage 110 et dans le sas de rhabillage 120 peuvent être suspendues au plafond de toile, et roulées avant le pliage de l'extension considérées. Ainsi, lors de la mis en service de la cellule 100, cette cloison se déroule et se met en place par l'effet de la seule gravité. Cela contribue à la facilité et à la rapidité de cette mise en service.

La contiguïté précitée entre les lignes de décontamination L1 et L2 n'est pas obligatoire. Les deux lignes de décontamination peuvent en effet être espacées l'une de l'autre, par exemple afin de mieux les isoler l'une de l'autre d'un point de vue sanitaire, en réduisant le risque d'éventuelles contaminations croisées. Une autre raison et un autre avantage d'un tel espacement suivant la direction de l'axe longitudinal X entre les lignes L1 et L2 peut être de permettre le respect de règles de bienséance s'appliquant à la population des personnes à décontaminer. De même, cela peut permettre de mieux respecter la pudeur et l'intimité desdites personnes à décontaminer.

Des résultats comparables ou similaires peuvent être obtenus pour le sas d'entrée 110 et pour le sas de sortie 120 avec des murs de séparation en dur, agencés entre les différents compartiments des deux lignes de décontamination L1 et L2. Il peut s'agir de cloisons fabriquées en panneaux sandwich dont les parements, c'est-à-dire les surfaces externes, peuvent être en Polyester non poreux, idéalement totalement lisse, afin d'être facilement décontaminable.

Afin d'assurer l'éclairage de la zone de travail à l'extérieur et autour de l'unité de décontamination chaque face latérale ainsi que sur la face arrière de la cellule 100 peut comprendre des luminaires, par exemple des luminaires à LED. Ces luminaires à LED peuvent être intégrées dans la traverse haute de la cellule, et notamment du module technique 101. Ils permettent d'éclairer de nuit la zone de travail autour de la cellule de décontamination. Ils peuvent être pilotés par un tableau de commande général 109 du module technique 101 de la cellule 100, accessible depuis l'extérieur, par exemple côté gauche de la cellule 100 comme représenté à la figure 3. Le fait que ce un tableau de commande général 109 soit accessible depuis l'extérieur de la cellule 100 permet la mise en service de l'éclairage extérieur dès l'arrivée sur la zone d'intervention, afin de faciliter la mise en service de nuit de l'unité. Ce un tableau de commande général 109 peut également servir à piloter la mise en place des vérins de stabilisation du châssis-porteur du camion porteur 200 avant le déploiement des extensions latérales 110 et 120 du module de douche 102. Si ce déploiement est, en tout ou en partie, commandé électriquement, alors cette commande peut avantageusement être également pilotée par l'opérateur via le tableau de commande général 109.

L'éclairage intérieur du module de douche 102 et le cas échéant de ses extensions 110 et 120, peut se faire par des luminaires (là encore de type LED, par exemple) placés dans chaque compartiment, soit à l'intérieur du module de douche 102 pour l'éclairage des compartiments de douche, soit à l'extérieur sur les parois externes dudit module 102 pour l'éclairage des extensions latérales 110 et 120. Le dimensionnement de l'éclairage intérieur est étudié de façon à avoir un confort de travail optimal sans créer de zones d'ombres. Les luminaires qui assurent l'éclairage intérieur peuvent être pilotés par une armoire électrique (non référencées sur les figures) associée à un pupitre de commande, qui sont compris dans le module technique 101 de la cellule 100.

Le module de douche 102 peut comprendre un indicateur lumineux, par exemple un indicateur bicolore rouge/vert, au niveau de chacune des portes d'entrée 108 et 109, à l'extérieur du module 102, et au niveau de chacune des portes de sortie 118 et 119, à l'intérieur du module 102, pour signifier aux personnes les moments où elles doivent entrer dans, ou sortir du module de douche, respectivement. D'autres indicateurs lumineux, notamment des indicateurs bicolores rouge/vert ou autres, peuvent aussi être montés entre les deux douches 134 et 136, ou 135 et 137 de chacun des compartiments de douche de la ligne de décontamination L1 et de la ligne de décontamination L2, respectivement. Ceci permet de fluidifier la circulation des personnes dans ces lignes de décontamination, et ainsi d'augmenter le débit de chaque ligne de décontamination. En effet, grâce notamment aux indicateurs lumineux, chaque personne sait à quel moment elle doit progresser le long de la ligne de décontamination qu'elle suit. Les indicateurs lumineux ci-dessus peuvent être pilotés par l'armoire électrique associée au pupitre de commande, qui sont compris dans le module technique 101 de la cellule 100.

L'armoire électrique et le pupitre de commande sont placés dans le compartiment technique 101 de la cellule 100. Ils rassemblent l'ensemble des commandes permettant de faire fonctionner tous les matériels nécessaires au bon fonctionnement de la cellule de décontamination 100, et notamment du module de douche 102.

Le fonctionnement des douches de l'unité peut avantageusement être automatique, ce qui est en faveur d'un débit plus important du flux de personnes traitées. La mise en marche/arrêt des douches est assuré par l'armoire électrique qui pilote les électrovannes montées sur la tuyauterie. Ces électrovannes permettent de contrôler l'arrivée d'eau aux portiques qui alimentent les pommeaux de douche 134, 135, 136 et 137. Toutefois, les deux lignes de décontamination L1 et L2 peuvent être opérées de manière indépendante l'une de l'autre. Ainsi, si une ligne est arrêtée pour quelque raison que ce soit, l'autre peut continuer à fonctionner. Par exemple, il y a une électrovanne par pommeau de douche, ce qui permet de contrôler la mise en marche/arrêt de chaque douche indépendamment des autres.

Des boutons de commandes et des voyants de signalisation du coffret électrique forment une interface homme-machine qui permet de contrôler les deux lignes de décontamination, soit en mode automatique soit en mode manuel par un opérateur 300 lorsqu'il est positionné dans le module technique 101, auquel il accède par le hayon 103. Le réglage du temps de fonctionnement des douches peut se faire par un menu affiché sur un écran, ce qui est simple d'utilisation. À tout moment pendant le fonctionnement de l'unité de décontamination 100, les temps programmés peuvent être modifiés sous la commande de l'opérateur 300. En parallèle et en lien avec le fonctionnement des douches, les indicateurs lumineux précités qui sont placés au-dessus de la porte d'entrée 108 ou 109 (à l'extérieur du module de douche), de la porte de sortie 118 ou 119 (à l'intérieur du module de douche), et au milieu des deux douches 134 et 136 ou des douches 135 et 137, la lumière s'allument en rouge ou en vert en fonction du fonctionnement des douches, afin de permettre aux personnes à décontaminer de progresser en rythme le long de la ligne de décontamination L1 ou L2, respectivement, qu'ils suivent.

Comme on l'aura compris, en plus des deux personnes qui peuvent simultanément se trouver dans le module de douche 102 comme il a été exposé plus haut en référence à la figure 6, deux personnes peuvent simultanément se trouver dans le sas d'entrée 110 (à raison d'une personne dans le compartiment de déshabillage de chaque ligne de décontamination L1 et L2, respectivement), et être en train de procéder à leur déshabillage. De plus, deux personnes peuvent simultanément se trouver dans le sas de sortie 120 (à raison d'une personne dans le compartiment de séchage et de rhabillage de chaque ligne de décontamination L1 et L2, respectivement), et être en train de se sécher et de s'habiller avec les vêtements à usage unique qu'elles trouvent dans ce sas de sortie 120.

En d'autres termes, à chaque étape du cycle de décontamination d'une personne, qui comprend quatre étapes à savoir le déshabillage, la douche de lavage, la douche de rinçage, et le rhabillage) ce sont en fait trois personnes qui sont impliquées simultanément dans les opérations de décontamination, et ce pour chacune des lignes de décontamination L1 ou L2 :
- une personne dans le sas d'entrée 110 en train de se déshabiller ;
- une personne dans le module de douche 102, sous la douche de lavage 134 ou 135, respectivement, ou ultérieurement à un poste en avant dans la ligne de décontamination, i.e., sous la douche de rinçage 136 ou 137, respectivement ; et enfin,
- une personne dans le sas de sortie 120 en train de se sécher et de se rhabiller.

A chaque cycle, et en rythme avec l'allumage rouge/vert des indicateurs lumineux, les personnes avancent dans la ligne L1 ou la ligne L2. Alors que la troisième personne ci-dessus sort du sas de sortie 120 par la porte 118a ou 119a, respectivement, et quitte donc l'unité de décontamination 100, elle y est remplacée par la personne qui sort du module de douche 102 par la sortie 118 ou 119, respectivement. Cette dernière personne est alors remplacée dans le module de douche 102 par la personne qui était dans le compartiment de déshabillage du sas d'entrée 110, et qui accède audit module de douche 102 par la porte 108 ou 109, respectivement, alors en outre qu'une nouvelle personne entre dans le sas d'entrée 110, par la porte 108a ou 109a, respectivement, et accède ainsi à l'unité de décontamination 100.

Comme on l'aura compris, le processus ci-dessus s'effectue en parallèle pour les deux lignes de décontamination L1 et L2, dans les deux compartiments de douche respectifs du module de douche 102, dans les deux compartiments de déshabillage respectifs de son extension 110 et dans les deux compartiments de séchage/rhabillage respectifs de l'extension son extension 120. Avantageusement, les deux lignes de décontamination L1 et L2 sont opérées en parallèle avec des moyens techniques communs qui sont présents dans le module technique 101.

En plus ou à la place des lignes de décontamination L1 et L2 du module de douche pour victimes valides (SV) qui ont été présentées jusqu'ici, un module de douche peut comprendre une ligne de décontamination L3 de victimes invalides (SI).

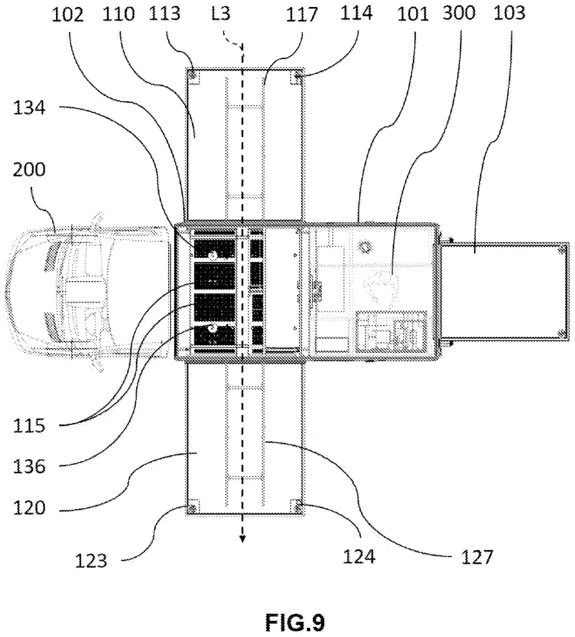

Un module de douche 102 avec une telle ligne de décontamination L3 va maintenant être décrit en référence aux **figures 9****,** 10 et **11****.** Ainsi qu'on l'appréciera cette ligne de décontamination L3 est capable d'être opérée, en mode dégradé, en tant que ligne de décontamination de personnes valides avec des moyens techniques qui sont présents dans le module technique 101.

Globalement, la conception du module de douche pour victimes invalides est identique à celle du module de douche pour victimes valides, excepté en ce qui concerne le détail des lignes de décontamination.

Dans le cas du module de douche pour personnes invalides, l'espace utilisé pour créer les deux lignes L1 et L2 pour personnes valides du module de douche 101 et de ses extensions latérales 110 et 120, est utilisé pour ne créer qu'une seule ligne de décontamination L3 qui est adaptée à la décontamination par voie humide des personnes invalides. Le module comprend aussi quelques équipements supplémentaires spécifiques à la ligne de décontamination L3 pour personnes invalides.

Ainsi, des supports hauts avec des rails de brancardage 117 et 127 sont disposés dans le sas d'entrée 110 et dans le sas de sortie 120, respectivement. Ces équipements sont réalisés, par exemple en inox. Le compartiment de douche 102 est équipé d'une table élévatrice à deux positions basse et haute, également réalisée en Inox, mue par un groupe hydraulique qui alimente un vérin double effet, ou une pompe manuelle en mode dégradé. La position basse de la table élévatrice vient de niveau avec les rails de brancardage 117 du sas d'entrée 110 et des rails de brancardage du sas de sortie 120. La base élévatrice vient en alignement opérationnel avec lesdits rails de brancardage 117 et 127. Ces trois groupes d'éléments de brancardage forment une ligne de brancardage suivant la direction de l'axe transversal Y.

De préférence, l'unité de décontamination est livrée avec trois brancards équipés de roulettes, permettant de faire passer une victime invalide à travers les différents compartiments de l'unité sans avoir à la porter : le compartiment de déshabillage dans le sas d'entrée 110, le compartiment de douche dans le module de douche 102, et le compartiment de séchage/rhabillage dans le sas de sortie 120.

La table élévatrice dans le module de douche 102 est munie de quatre pans inclinés, l'un à chacune des extrémités des deux rails qu'elle comporte pour recevoir le brancard. Ces pans inclinés comporte une zone de butée permettant l'arrêt en translation du brancard à roulettes. Lorsqu'un brancard avec une victime invalide dessus a été amené en position sur la table élévatrice et immobilisé en translation par coopération des roulettes contre la butée des pans inclinés, la table est élevée dans sa position haute. Ainsi, la victime se trouve à hauteur pour recevoir une douche par un opérateur.

Sur le circuit d'alimentation en eau des portiques de lavage et de rinçage un piquage équipé d'une vanne 1/4T et d'un raccord rapide est présent. Il permet de connecter deux douchettes à mains pour la décontamination des victimes invalides. Avantageusement, la toile du brancard peut être perforée afin que l'eau puisse s'écouler à travers.

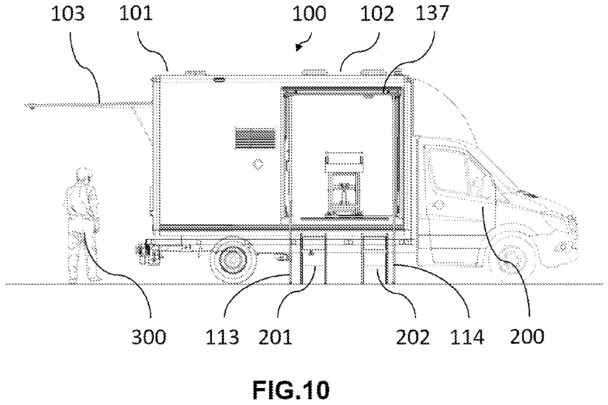

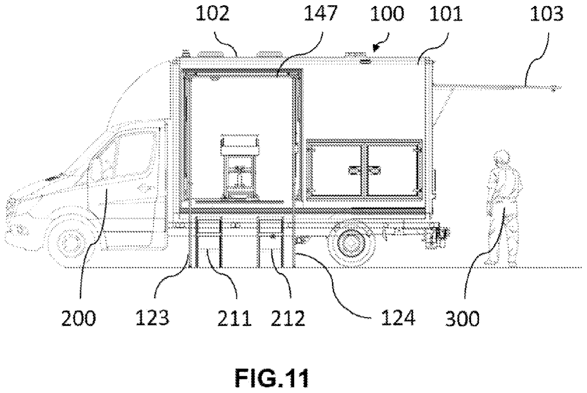

En référence en outre aux **figures 10** et **11****,** l'accès et la sortie du module de douche 102 se font par des rideaux souples 137 et 147, du côté droit (côté du sas d'entrée 110) et du côté gauche (côté du sas de sortie 120), respectivement. Ces rideaux souples 137 et 147 sont par exemple réalisés en PVC. Ces rideaux peuvent être équipés d'une fenêtre de chaque côté. La partie centrale du rideau peut se relever lors de l'entrée ou la sortie de la victime du compartiment douche.

L'entrée dans le compartiment déshabillage et la sortie du compartiment rhabillage se font par une porte placée aux extrémités des extensions. De préférence, la porte fait toute la largeur de l'extension, à savoir 1800 mm dans l'exemple, afin de faciliter le passage du brancard. La porte se ferme grâce à des fermetures à glissières. Elle peut être maintenue en position haute, ou au niveau de la hauteur des rails 107 et 117 des supports brancards.

De manière avantageuse, le module de douche 102 pour victimes invalides peut être utilisé en mode dégradé pour une seule ligne de décontamination de victime valides. C'est la présence de la table élévatrice qui empêche de l'utiliser pour deux lignes de décontamination pour victime valides en parallèle. En effet, la table élévatrice est montée sur glissière et se range contre la cloison avant du module de douche 102, du côté du conducteur du véhicule 200.

L'unité de décontamination est autonome dans son fonctionnement. A cet effet, elle comprend un groupe électrogène qui permet de fournir l'énergie électrique nécessaire au fonctionnement des différents équipements. Également, elle comprend une citerne d'eau propre pour l'alimentation des douches, et au moins un bas de récupération des eaux usées.

En référence de nouveau à la **figure 2** et à la **figure 3****,** notamment, le groupe électrogène 105 peut être placé sur le côté du module technique 101, à savoir le côté gauche dans l'exemple représenté. Il reste ainsi installé à demeure pendant le fonctionnement de l'unité. Son alimentation en carburant est réalisée depuis un réservoir de gasoil commun (non représenté), par exemple de capacité égale à 100 litres, du module technique 101.

Il peut s'agir, par exemple, d'un groupe électrogène de type EPS6000DE HA/LS. Un tel groupe offre une puissance maximum de 5.5 kV, avec une puissance continue de 5 kVA, équipé d'une prise 230V - 22A. L'alternateur est un alternateur Sincro EK2MCT EK6 6 kVA SAEJ609B, délivrant un courant de fréquence égale à 60 Hz. Le moteur thermique est un moteur HATZ 1B40, 1-cylindre, 462cm³, 3000 tpm, refroidi par air.

L'homme du métier appréciera que le groupe électrogène ci-dessus n'est qu'un exemple, et que en pratique le groupe électrogène utilisé doit être choisi en fonction des caractéristiques techniques de l'application envisagée. De préférence, on prévoit qu'une réserve de puissance d'au moins 30% soit disponible, afin de pouvoir connecter des équipements annexes utiles au fonctionnement de l'unité de décontamination, le cas échéant.

La connexion du groupe électrogène à la cellule se fait par une prise placée dans le local technique. Cela permet de pouvoir connecter la cellule à un réseau de distribution électrique 220V / 60Hz extérieur en mode parking, à la place du groupe électrogène, lorsque cela est possible. Le groupe électrogène est alors utilisé comme source d'énergie électrique de secours, pour assurer la continuité opérationnelle même en cas de coupure passagère de cette alimentation électrique par un réseau de distribution électrique.

Pour la récupération des eaux usées contaminées, l'unité de décontamination mobile 100 comprend un ou plusieurs réservoirs souples annexes, comme le réservoir 420 montré à la **figure 3****.**

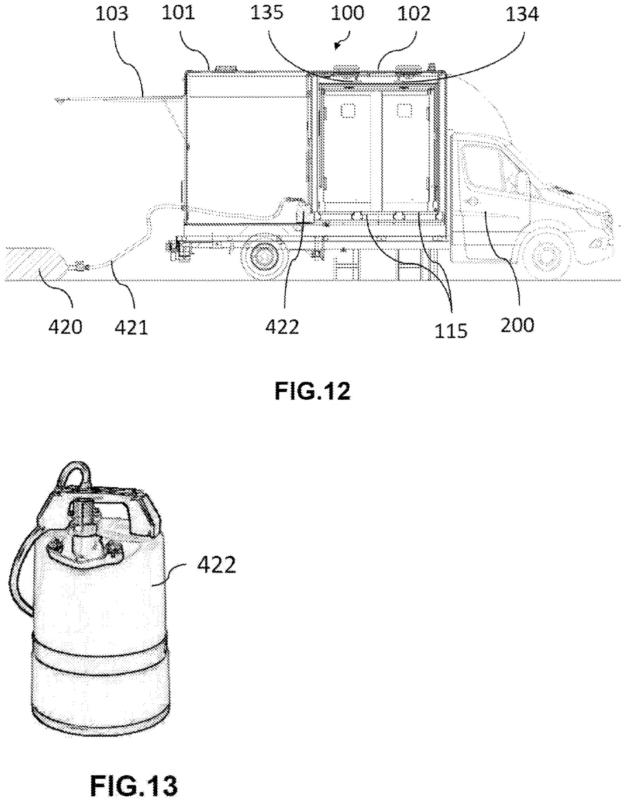

En référence à la **figure 12****,** le bac tampon qui est placé sous le plancher en caillebotis 115 du module de douche 102, peut comprendre un profil en V avec une pente dirigée vers le module technique 101. Ce dernier comprend une pompe 422, par exemple une pompe « serpillère » (i.e., une pompe submersible qui peut être posée sur la surface à aspirer) comme représentée à la **figure 13****,** qui est adaptée et agencée pour aspirer les eaux usées dans le bac tampon. Les eaux usées sont évacuées vers le réservoir souple 420 de récupération, par l'intermédiaire d'une conduite souple 421.

Le (ou les) réservoir(s) souple(s) sont mise en place, ainsi par l'opérateur 300 lors de l'installation et la mise en service de l'unité de décontamination. Le réservoir souple est remplacé par un autre à chaque fois qu'il est plein. Etant placé à l'extérieur de l'unité de décontamination 100, de préférence à l'arrière de ladite unité, cette opération de remplacement est facile de même que l'enlèvement des réservoirs pleins. Surtout, ces opérations n'interfèrent pas avec la circulation des personnes à travers le module de douche 102.

Dans un exemple, l'unité de décontamination mobile est équipée de deux réservoirs souples de 3000 litres chacun, réalisés en toile polyester enduite de PVC sur les 2 faces, ayant une densité de 1100g/m². Ils peuvent être équipées de brides en polypropylène, et d'une visserie inox. Pour le raccordement à la conduite souple 421, les réservoirs sont par exemple équipés d'une vanne 1/4T et d'un raccord pompier DN45.

La pompe 422 est alimentée électriquement par un bouton Marche/Arrêt placé sur le tableau de commande général, sa mise en service se fait automatiquement dès la présence d'eau dans le bac tampon et s'arrête dès qu'il n'y a plus d'eau dans celui-ci. Un fonctionnement en marche forcée peut être effectué pour vidanger la totalité du bac tampon.

Le débit de la pompe utilisée est 6 m³/h. La granulométrie des particules solides pouvant être aspirées est égale à 6 mm. De préférence, le débit de la pompe est largement dimensionné, afin de de garantir que l'eau des douches ne peut pas déborder du bac tampon lors du fonctionnement de l'unité de décontamination à pleine charge.

Dans un exemple correspondant à la figure 13, la pompe submersible est à alimentation électrique 230 V - - 50 Hz, de puissance égale à 0.4 kW. Son débit maximum est de 10 m³/h. La pompe est capable d'aspirer l'eau à partir de 1 mm de hauteur. Sa puissance électrique est de 480 W.

Dans des modes de réalisation, l'alimentation en eau propre de la cellule de décontamination 100 peut se faire à partir d'une citerne externe 410 comprise dans la remorque qui a déjà été présentée plus haut en référence à la **figure** 3 à laquelle on se réfère à nouveau ici.

Cette citerne permet de stocker et transporter l'eau nécessaire au fonctionnement de la cellule de décontamination sur n'importe quel site d'intervention. Avantageusement, elle peut être remplacée, lorsqu'elle est vide, par une autre citerne comparable qui peut être apportée sur site par un autre véhicule. Et ceci, sans avoir besoin de déplacer l'unité de décontamination, et donc sans interruption de son fonctionnement.

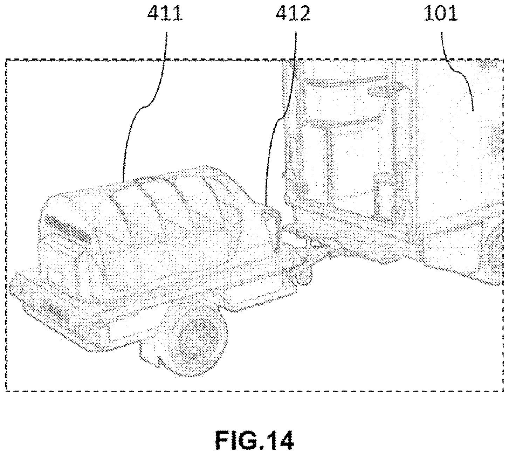

Comme montré à la **figure 14****,** la citerne est de préférence compartimentée, afin de réduire le balourd lors des déplacements. Sa capacité peut être de 1000 litres, par exemple, qui procure à l'unité de décontamination une autonomie en eau égale à 6 heures environ.

La remorque peut être équipée d'une pompe de transfert 412 adaptée pour le transfert de l'eau vers les circuits d'alimentation des douches de la cellule de décontamination. La pompe 412 peut être électriquement alimentée par le groupe électrogène du module technique 101, à l'aide d'un câble d'alimentation électrique associé audit module technique. En variante, la pompe peut être comprise dans le module technique 101.

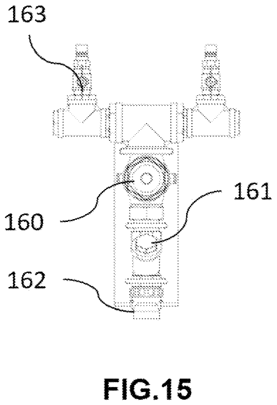

En référence à la **figure 15****,** le système d'alimentation en eau de l'unité de décontamination mobile comprend en outre un régulateur de pression d'eau 160. Le régulateur peut être adapté pour réguler la pression d'eau dans le réseau d'alimentation à une valeur réglable entre 0 et 6 bars, par exemple.

Le régulateur de pression d'eau 160 peut être équipé d'un filtre à boue 161, et d'un manomètre 0/10 bars en acier et à lecture directe (non représenté). Par exemple le filtre à boue 161 peut être adapté pour filtrer toutes les impuretés dans l'eau d'un diamètre supérieur à 500 microns.

Le régulateur de pression d'eau 160 peut être équipé en entrée d'un raccord 162, par exemple un raccord pompier DN45, permettant de se connecter sur un camion pompier, un hydrant ou un réseau d'eau local. Ainsi, l'autonomie en eau de l'unité de décontamination n'est plus limitée à la capacité de la citerne, dès lors qu'une autre source d'alimentation en eau est disponible à proximité.

En outre, il peut être équipé en sortie d'une vanne quart-de-tour (¼ T) 163 permettant à tout moment de couper l'alimentation en eau de l'unité de décontamination.

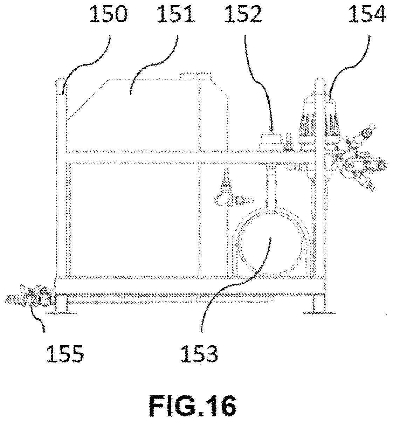

En référence à la **figure 16****,** le système d'alimentation en eau de l'unité de décontamination mobile 100 permet d'alimenter en eau mitigée l'ensemble des douches. Il comprend différents matériels installés dans le module technique. De tels matériels peuvent être montés sur un châssis tubulaire 150, par exemple en Inox qui est fixé dans le module technique et y reste à demeure lors du fonctionnement de l'unité de décontamination mobile.

Les éléments qui composent le système d'alimentation en eau sont les suivants :
- une chaudière instantanée 151, ayant par exemple une puissance de 57Kw et un débit de 35 l/mn fonctionnant au gasoil, les gaz d'échappement étant évacués en toiture du module technique. Son alimentation en gasoil peut se faire par une canalisation souple raccordée à un réservoir central, d'une capacité de 100 litres par exemple, qui est placé dans la le module technique ;
- un mitigeur de température 152, permettant de régler la température de l'eau dans une plage située entre 15°C et 50°C. Le réglage de la température se fait par exemple par une molette graduée à lecture directe. La température d'eau chaude maximum est par exemple de 85 °C ;
- un ballon tampon 153, par exemple d'une capacité de 20 litres environ, réalisé par exemple en Inox, et qui permet d'assurer une température homogène sur les quatre douches du module de douche envisagé dans la présente description et tel que décrit plus haut ;
- une pompe mélangeuse 154, par exemple une pompe à débit proportionnel avec une plage de réglage de 0,2 à 2%, sous une pression réglable de 0,5 à 6 bars. Le débit opérationnel peut être compris entre 10 l/mn et 3 m³/h. La une pression de service recommandée est comprise entre 2 et 4 bars. Cette pompe est adaptée pour injecter la solution de décontamination dans l'eau mitigée. Le réglage se fait par exemple une molette graduée à lecture directe. Les éléments constituant la pompe mélangeuse permettent avantageusement le passage de produits corrosifs. A cet effet, le corps et la cloche du mélangeur peuvent par exemple être réalisés en PVDF. Elle peut être équipée d'un kit visqueux permettant le passage de produits de décontamination dont la viscosité peut aller jusqu'à 800 cps à 20°C ; et,
- des vannes de vidange 155, disposées en bas du châssis, permettant la vidange, notamment, du corps de chauffe de la chaudière 151, du ballon 153, et de la pompe 154.

Les équipements ci-dessus procure une régulation et une stabilité de la température de l'eau satisfaisantes quelles que soient les variations de pression (avec un maximum de 1,5 bar), de température aux entrées du mitigeur, et du débit (dans une plage comprise entre 3 et 42 l/min).

La présente invention a été décrite et illustrée dans la présente description détaillée et dans les figures des dessins annexés, dans des formes de réalisation possibles. La présente invention ne se limite pas, toutefois, aux formes de réalisation présentées. D'autres variantes et modes de réalisation peuvent être déduits et mis en oeuvre par la personne du métier à la lecture de la présente description et des dessins annexés, tant que ceux-ci restent dans le champ d'application des revendications annexées.

En particulier, l'homme du métier appréciera que le nombre de lignes de décontamination n'est pas limité à deux comme l'exemple considéré dans la présente description. De plus, on peut prévoir une combinaison d'un ou plusieurs modules de douche pour personnes valides comme le module des figures 6, 7 et 8, et d'un ou plusieurs modules de douche pour personnes invalides comme le module des figure 9, 10 et 11, dont le fonctionnement est assuré par un unique module technique comme le module 101, dimensionné pour cela.

Également, bien l'unité de décontamination mobile 100 décrite ici soit particulièrement adaptée pour son installation à demeure sur le châssis d'un véhicule-porteur comme représenté, d'autres applications sont possibles. En variante en effet, l'unité de décontamination mobile peut être fournie en la forme d'un conteneur ou d'un demi-conteneur formant cellule prête à l'emploi qui peut être déposée par un camion-grue qui ne reste pas sur le site d'intervention. Elle comprend alors, sur le dessus, des crochets de levage et/ou, sur le dessous, des trous pour les fourches d'un chariot élévateur. Cette configuration est adaptée aux applications dans lesquelles l'unité de décontamination fonctionne de façon plus durable, par exemple dans le cadre d'un chantier, comme un chantier de décontamination d'un site industriel.

Dans le présent exposé, le terme "comprendre" ou "comporter" n'exclut pas d'autres éléments ou d'autres étapes. Un seul processeur ou plusieurs autres unités peuvent être utilisées pour mettre en oeuvre l'invention. Les différentes caractéristiques présentées peuvent être avantageusement combinées. Leur présence dans des parties différentes, n'excluent pas cette possibilité. Les signes de référence ne sauraient être compris comme limitant la portée de l'invention.

## Revendications

1. Unité de décontamination mobile (100) pour la décontamination de personnes par voie humide, ayant la forme générale d'un parallélépipède rectangle avec un axe longitudinal (X) et un axe transversal (Y), **caractérisée en ce qu'**elle comprend :
- un module technique (101) et au moins un module de douche (102), adjacents l'un à l'autre suivant la direction de l'axe longitudinal ;
- une première extension escamotable (110) du module de douches suivant la direction de l'axe transversal, d'un côté dudit module de douche, et une seconde extension escamotable (120) du module de douche suivant ladite direction de l'axe transversal, de l'autre côté dudit module de douche, les extensions 110 et 120 sont formées de toiles en PVC, le module de douche ainsi que sa première extension et sa seconde extension comprenant au moins une ligne de décontamination (L1,L2,L3) de personnes pour la circulation, suivant la direction de l'axe transversal, de personnes à décontaminer par douchage dans le module de douche, dans laquelle le module de douche est équipé d'une douche de décontamination et d'une douche de rinçage pour chaque ligne de décontamination, comprenant chacune un pommeau de douche avec un circuit d'alimentation alimentée par une solution de décontamination et avec un autre circuit d'alimentation alimenté par de l'eau claire, respectivement.

2. Unité de décontamination selon la revendication 1, dans laquelle le module de douche ainsi que sa première extension et/ou sa seconde extension comprennent au moins deux lignes de décontamination (L1,L2) qui sont adaptées pour être opérées en parallèle avec des moyens techniques communs compris dans le module technique (101).

3. Unité de décontamination selon l'une quelconque la revendication 2, dans laquelle le module de douche comprend une paroi amovible s'étendant suivant la direction de l'axe transversal afin de séparer deux compartiments de douche du module de douche, lesdits compartiments de douche appartenant à la première ligne de décontamination (L1) et à la seconde ligne de décontamination (L2), respectivement,
et dans laquelle la première extension (110) et/ou la seconde extension (120) du module de douche comprennent chacune une paroi s'étendant suivant la direction de l'axe transversal afin de séparer ladite extension en deux compartiments, lesdits compartiments appartenant à la première ligne de décontamination (L1) et à la seconde ligne de décontamination (L2), respectivement.

4. Unité de décontamination selon l'une quelconque des revendications 1 à 3, comprenant un unique bac tampon qui est placé sous un plancher en caillebotis du module de douche, et comprenant en outre une pompe qui est placée dans le module technique et qui est adaptée pour aspirer les eaux usées dans le bac tampon et les évacuer vers un ou plusieurs réservoirs souples de récupération des eaux usées qui peuvent être placés à l'extérieur de l'unité de décontamination.

5. Unité de décontamination selon la revendication 4, dans laquelle le fond du bac tampon est à profil en V, avec une pente dirigée vers la pompe.

6. Unité de décontamination selon l'une quelconque des revendications 1 à 5, dans laquelle la première extension et/ou la seconde extension du module de couche sont en toile, et sont dépliables et repliables avec des éléments d'armature et des poteaux par lesquels la toile est soutenue, ladite toile, lesdits éléments d'armature est lesdits poteux étant escamotables dans la paroi du module de douche.

7. Unité de décontamination selon l'une quelconque des revendications 1 à 6, dans laquelle le module technique comprend un ventilateur raccordé à une gaine de diffusion d'air (142) débouchant dans la seconde extension (120), et adapté pour assurer un débit d'air assurant un balayage d'air en surpression depuis la seconde extension (120) vers la première extension (110) à travers le module de douche (102).

8. Unité de décontamination selon l'une quelconque des revendications 1 à 7, dans laquelle le fonctionnement du module de douches de l'unité est automatique, et rythmé par des indicateurs lumineux placé au-dessus de la porte d'entrée, de la porte de sortie et au milieu des deux compartiments de douche, la lumière s'allumant rouge ou verte en fonction du fonctionnement des douches.

9. Procédé d'utilisation d'une unité de décontamination selon la revendication 8, dans lequel le fonctionnement de douches du module de douche est automatique, et est commandé en rythme avec l'allumage d'indicateurs lumineux adaptés pour signaler aux personnes valides leur progression dans la ligne de décontamination pour personnes valides.

## Patentansprüche

1. Mobile Dekontaminationseinheit (100) zur Nassdekontamination von Personen, die die allgemeine Form eines rechteckigen Parallelepipeds mit einer Längsachse (X) und einer Querachse (Y) aufweist, **dadurch gekennzeichnet, dass** sie Folgendes umfasst:
- mindestens ein technisches Modul (101) und mindestens ein Duschmodul (102), die einander entlang der Richtung der Längsachse benachbart sind;
- mindestens eine erste, entlang der Richtung der Querachse von einer Seite des Duschmoduls einziehbare Erweiterung (110) des Duschmoduls, und eine entlang der Richtung der Querachse von der anderen Seite des Duschmoduls zweite einziehbare Erweiterung (120) des Duschmoduls, wobei die Erweiterungen 110 und 120 aus Geweben aus PVC gebildet sind, wobei das Duschmodul sowie seine erste Erweiterung als auch seine zweite Erweiterung entlang der Richtung der Querachse mindestens eine Personen-Dekontaminationsstrecke (L1, L2, L3) für die Zirkulation von durch Duschen in dem Duschmodul zu dekontaminierenden Personen umfassen, wobei das Duschmodul für jede Dekontaminationsstrecke mit einer Dekontaminationsdusche und eine Spüldusche, jeweils umfassend einen Duschkopf mit einem Zufuhrkreislauf, der durch eine Dekontaminationslösung gespeist wird, und mit einem Zufuhrkreislauf ausgestattet ist, der durch klares Wasser gespeist wird.

2. Dekontaminationseinheit nach Anspruch 1, wobei das Duschmodul sowie seine erste Erweiterung und/oder seine zweite Erweiterung mindestens zwei Dekontaminationsstrecken (L1, L2) umfassen, die angepasst sind, um mit gemeinsamen, in dem technischen Modul (101) umfassten Mitteln parallel betrieben zu werden.

3. Dekontaminationseinheit nach einem von Anspruch 2, wobei das Duschmodul eine abnehmbare Wand umfasst, die sich entlang der Richtung der Querachse erstreckt, um zwei Kompartimente der Dusche des Duschmoduls zu trennen, wobei die Kompartimente der Dusche jeweils zu der ersten Dekontaminationsstrecke (L1) und zu der zweiten Dekontaminationsstrecke (L2) gehören,
und wobei die erste Erweiterung (110) und/oder die zweite Erweiterung (120) des Duschmoduls jede eine Wand umfassen, die sich entlang der Richtung der Querachse erstrecken, um die Erweiterung in zwei Kompartimente zu teilen, wobei die Kompartimente jeweils zu der ersten Dekontaminationsstrecke (L1) und zu der zweiten Dekontaminationsstrecke (L2) gehören.

4. Dekontaminationseinheit nach einem der Ansprüche 1 bis 3, umfassend einen einzigen Pufferbehälter, der unter einem Gitterrostboden des Duschmoduls platziert ist, und weiter umfassend eine Pumpe, die in dem technischen Modul platziert ist und angepasst ist, um das Abwasser in dem Pufferbehälter abzusaugen und es in Richtung eines oder mehrerer flexibler Tanks, die außerhalb der Dekontaminationseinheit platziert werden können, zur Wiedergewinnung des Abwassers zu evakuieren.

5. Dekontaminationseinheit nach Anspruch 4, wobei der Boden des Pufferbehälters in einem V-Profil vorliegt, mit einem in Richtung der Pumpe gerichteten Gefälle.

6. Dekontaminationseinheit nach einem der Ansprüche 1 bis 5, wobei die erste Erweiterung und/oder die zweite Erweiterung des Schichtmoduls aus Gewebe bestehen und mit Verstärkungselementen und Pfosten, mit denen das Gewebe gestützt wird, klapp- und faltbar sind, wobei das Gewebe, die Verstärkungselemente und die Pfosten in die Wand des Duschmoduls einziehbar sind.

7. Dekontaminationseinheit nach einem der Ansprüche 1 bis 6, wobei das technische Modul einen mit einem Luftdiffusionskanal (142), der in der zweiten Erweiterung (120) mündet, verbundenen Ventilator umfasst und angepasst ist, um einen Luftstrom zu gewährleisten, der eine Überdruckluftspülung ab der zweiten Erweiterung (120) in Richtung der ersten Erweiterung (110) durch das Duschmodul (102) hindurch gewährleistet.

8. Dekontaminationseinheit nach einem der Ansprüche 1 bis 7, wobei der Betrieb des Duschmoduls der Einheit automatisch ist und durch über der Eingangstür, der Ausgangstür und in der Mitte der zwei Duschkompartimente platzierten Leuchtmeldern getaktet wird, wobei das Licht abhängig von dem Betrieb der Duschen rot oder grün aufleuchtet.

9. Verwendungsverfahren einer Dekontaminationseinheit nach Anspruch 8, wobei der Betrieb der Duschen des Duschmoduls automatisch ist und im Takt mit dem Aufleuchten der Leuchtmelder gesteuert wird, die angepasst sind, um validen Personen ihren Fortschritt in der Dekontaminationsstrecke für valide Personen zu signalisieren.

## Claims

1. Mobile decontamination unit (100) for wet decontamination of persons, having a generally rectangular parallelepiped shape with a longitudinal axis (X) and a transverse axis (Y), **characterised in that** it comprises:
- a technical module (101) and at least one shower module (102), which are adjacent to one another in the direction of the longitudinal axis;
- a first extension (110) of the shower module, which can be retracted in the direction of the transverse axis, on one side of the shower module and second extension (120) of the shower module, which can be retracted in the direction of the transverse axis, on the other side of the shower module, the extensions 110 and 120 being made of PVC canvas, the shower module and the first and second extension thereof comprising at least one person-decontamination line (L1, L2, L3) for the circulation of persons to be decontaminated by showering in the shower module in the direction of the transverse axis, wherein the shower module is equipped with a decontamination shower and a rinsing shower for each decontamination line, each comprising a shower head with a supply circuit fed with a decontamination solution and with another supply circuit fed with clear water, respectively.

2. Decontamination unit according to claim 1, wherein the shower module as well as its first extension and/or its second extension comprise at least two decontamination lines (L1, L2) which are suitable for being operated in parallel with common technical means comprised in the technical module (101).

3. Decontamination unit according to any one claim 2, wherein the shower module comprises a removable wall extending in the direction of the transverse axis to separate two shower compartments of the shower module, said shower compartments belonging to the first decontamination line (L1) and the second decontamination line (L2), respectively,
and wherein the first extension (110) and/or the second extension (120) of the shower module each comprise a wall extending in the direction of the transverse axis to separate said extension into two compartments, said compartments belonging to the first decontamination line (L1) and to the second decontamination line (L2), respectively.

4. Decontamination unit according to any one of claims 1 to 3, comprising a single buffer tank which is located under a grated floor of the shower module, and further comprising a pump which is located in the technical module and is suitable for drawing waste water into the buffer tank and discharging it to one or more flexible waste water collection tanks which may be located outside the decontamination unit.

5. Decontamination unit according to claim 4, wherein the bottom of the buffer tank is V-shaped, with a slope directed towards the pump.

6. Decontamination unit according to any one of claims 1 to 5, wherein the first extension and/or the second extension of the layer module are made of canvas, and are unfoldable and collapsible with frame elements and posts by which the canvas is supported, said canvas, frame elements, and posts being retractable into the wall of the shower module.

7. Decontamination unit according to any one of claims 1to 6, wherein the technical module comprises a fan connected to an air diffusion duct (142) opening into the second extension (120) and suitable for providing an air flow ensuring a sweep of overpressure air from the second extension (120) to the first extension (110) through the shower module (102).

8. Decontamination unit according to any one of claims 1to 7, wherein the operation of the shower module of the unit is automatic and timed by light indicators located above the entrance door, the exit door and in the middle of the two shower compartments, with the light turning red or green depending on the operation of the showers.

9. Method for using a decontamination unit according to claim 8, wherein the operation of showers of the shower module is automatic, and is controlled in rhythm with the illumination of light indicators suitable for signalling to able-bodied persons their progress through the able-bodied person decontamination line.
